# EUROPEAN PATENT APPLICATION

(11) **EP 3 636 153 A1**
(43) Date of publication of application: **15.04.2020**
(21) Application number: 19167039.7
(22) Date of filing: 03.04.2019
(51) Int. Cl.: A61B 5/11, A61B 5/00, A61B 5/107

(54) **CYCLING-POSTURE ANALYZING SYSTEM AND METHOD**

(30) Priority: 08.10.2018 TW 107135396
(71) Applicant: Institute for Information Industry, Taipei (TW)
(72) Inventor: Lin, Tse-Yu, 220 New Taipei City (TW); Chou, Yin-Yu, 950 Taitung City, Taitung County (TW); Wei, Shih-Yao, 116 Taipei City (TW)
(74) Representative: Michalski Hüttermann & Partner Patentanwälte mbB

(57) **Abstract**

A cycling-posture analyzing system and method are provided. The cycling-posture analyzing system includes a plurality of motion sensors, a pressure sensor and an electronic device. The plurality of motion sensors are disposed on a human body and are configured to detect a plurality of pieces of motion information. The pressure sensor is disposed at a plantar aspect of the human body and is configured to detect pressure information. The electronic device is configured to receive the plurality of pieces of motion information from the plurality of motion sensors, receive the pressure information from the pressure sensor, and use the plurality of pieces of motion information and the pressure information to determine cycling-posture type information according to a cycling-posture identification model.

## Description

### CROSS-REFERENCES TO RELATED APPLICATIONS

Not applicable.

### BACKGROUND OF THE INVENTION

### Field of the Invention

The present invention relates to a cycling-posture analyzing system and a cycling-posture analyzing method; and more particularly, the present invention uses a cycling-posture identification model to analyze data detected by motion sensors and a pressure sensor so as to determine cycling-posture type information of a bicycle.

### Descriptions of the Related Art

With the popularity of leisure activities, more and more people have joined the bicycle sport. People are turning their attention to know whether their cycling-posture is correct or not, so as to expect for achieving better efficiency and performance on bicycling or prevent the sports injuries caused by incorrect posture.

Currently, the analysis for the cycling-posture of bicyclists is often introduced by indoor image capturing, and movements of various parts of a human body are captured by a camera to analyze whether the cycling-posture is correct. However, due to the limitation of the image-capturing equipment, such kind of measuring manner cannot be promoted to measure the actual cycling state outdoors. Moreover, another method for analysis that is often used is measuring muscle states by sensors, and thereby obtain a measured surface electromyography (sEMG). However, human body could sweat copiously during the cycling, and such influences the adhesion ability and the detection precision of electrodes of the sensors, so it is hard to obtain precise analysis.

Accordingly, an urgent need exists in the art to provide a measuring method adapted for use in outdoor cycling to analyze the cycling-posture so as to provide riders with relevant suggestions, thereby improving the cycling performance of the riders.

### SUMMARY OF THE INVENTION

To solve the aforesaid problem, the present invention provides a cycling-posture analyzing system and a cycling-posture analyzing method.

The present invention provides a cycling-posture analyzing system, which comprises a plurality of motion sensors, a pressure sensor and an electronic device. The plurality of motion sensors are disposed on a human body and configured to detect a plurality of pieces of motion information. The pressure sensor is disposed at a plantar aspect of the human body and configured to detect pressure information. The electronic device further comprises a transceiver and a processor, and the processor is electrically connected to the transceiver. The transceiver is configured to receive the plurality of pieces of motion information from the plurality of motion sensors and the pressure information from the pressure sensor. The processor is configured to determine cycling-posture type information according to the plurality of pieces of motion information and the pressure information and on the basis of a cycling-posture identification model.

Moreover, the present invention provides a cycling-posture analyzing system, which comprises a plurality of motion sensors, a pressure sensor and an electronic device. The plurality of motion sensors are disposed on a human body and configured to detect a plurality of pieces of motion information. The pressure sensor is disposed at a plantar aspect of the human body and configured to detect pressure information. The electronic device further comprises a transceiver. The transceiver is configured to: receive the plurality of pieces of motion information from the plurality of motion sensors and receive the pressure information from the pressure sensor; transmit the plurality of pieces of motion information and the pressure information to a cloud computing system so that the cloud computing system determines cycling-posture type information, muscle-group usage information and feedback information according to the plurality of pieces of motion information and the pressure information; and receive the cycling-posture type information, the muscle-group usage information and the feedback information from the cloud computing system.

Additionally, the present invention provides a cycling-posture analyzing method for a cycling-posture analyzing system. The cycling-posture analyzing system comprises a plurality of motion sensors, a pressure sensor and an electronic device. The cycling-posture analyzing method comprises: detecting, by the plurality of motion sensors, a plurality of pieces of motion information, the plurality of motion sensors being disposed on a human body; detecting, by the pressure sensor, pressure information, the pressure sensor being disposed at a plantar aspect of the human body; receiving, by the electronic device, the plurality of pieces of motion information from the plurality of motion sensors and the pressure information from the pressure sensor; and determining, by the electronic device, cycling-posture type information according to the plurality of pieces of motion information and the pressure information on the basis of a cycling-posture identification model.

Furthermore, the present invention provides a cycling-posture analyzing method for a cycling-posture analyzing system. The cycling-posture analyzing system comprises a plurality of motion sensors, a pressure sensor and an electronic device. The cycling-posture analyzing method comprises: detecting, by the plurality of motion sensors, a plurality of pieces of motion information, the plurality of motion sensors being disposed on a human body; detecting, by the pressure sensor, pressure information, the pressure sensor being disposed at a plantar aspect of the human body; receiving, by the electronic device, the plurality of pieces of motion information from the plurality of motion sensors and the pressure information from the pressure sensor; transmitting, by the electronic device, the plurality of pieces of motion information and the pressure information to a cloud computing system so that the cloud computing system determines cycling-posture type information, muscle-group usage information and feedback information according to the plurality of pieces of motion information and the pressure information; and receiving, by the electronic device, the cycling-posture type information, the muscle-group usage information and the feedback information from the cloud computing system.

The detailed technology and preferred embodiments implemented for the subject invention are described in the following paragraphs accompanying the appended drawings for people skilled in this field to well appreciate the features of the claimed invention.

### BRIEF DESCRIPTION OF THE DRAWINGS

**FIG. 1** is a schematic view depicting a cycling-posture analyzing system according to a first embodiment;
**FIG. 2** is a schematic view depicting angular information and pressure information in a cycling-posture analyzing system according to a second embodiment;
**FIG. 3A** is a schematic view depicting an electronic device according to a fourth embodiment;
**FIG. 3B** is a schematic view depicting an identification model according to the fourth embodiment;
**FIG. 4A** is a schematic view depicting an electronic device according to a fifth embodiment;
**FIG. 4B** is a schematic view depicting a feedback model according to the fifth embodiment;
**FIG. 5** is a schematic view depicting an electronic device and a cloud computing system according to a sixth embodiment;
**FIG. 6** is a schematic view depicting a cycling-posture analyzing method according to a seventh embodiment;
**FIG. 7** is a schematic view depicting a cycling-posture analyzing method according to an eighth embodiment;
**FIG. 8** is a schematic view depicting a cycling-posture analyzing method according to a ninth embodiment;
**FIG. 9** is a schematic view depicting a cycling-posture analyzing method according to a tenth embodiment;
**FIG. 10** is a schematic view depicting a cycling-posture analyzing method according to an eleventh embodiment; and
**FIG. 11** is a schematic view depicting a cycling-posture analyzing method according to a twelfth embodiment.

### DESCRIPTION OF THE PREFERRED EMBODIMENT

In the following description, the present invention will be explained with reference to embodiments thereof. The present invention relates to a cycling-posture analyzing system and a cycling-posture analyzing method. It shall be appreciated that, these embodiments of the present invention are not intended to limit the present invention to any specific environment, applications or particular implementations described in these embodiments. Therefore, description of these embodiments is only for purpose of illustration rather than to limit the present invention, and the scope claimed by this application shall be governed by the claims. Besides, in the following embodiments and the attached drawings, elements unrelated to the present invention are omitted from depiction; and dimensional relationships among individual elements in the attached drawings are provided only for illustration, but not to limit the actual scale.

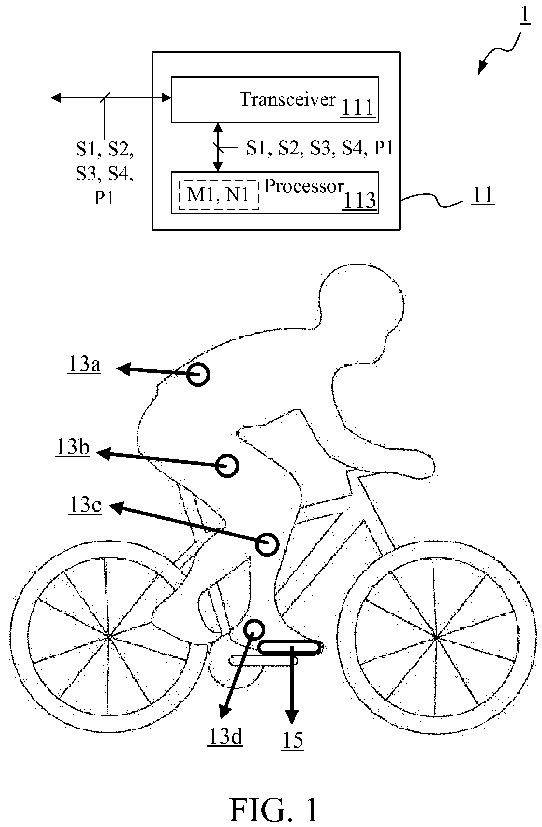

Please refer to **FIG. 1** for a first embodiment of the present invention. **FIG. 1** is a schematic view depicting a cycling-posture analyzing system **1.** Herein, the cycling-posture analyzing system **1** comprises an electronic device **11,** a plurality of motion sensors **13a** to **13d** and a pressure sensor **15,** and the configuration thereof is as shown in **FIG. 1****.** The operation of the cycling-posture analyzing system will be described hereinafter.

Specifically, the electronic device **11** comprises a transceiver **111** and a processor **113** which are electrically connected with each other. Four motion sensors **13a, 13b, 13c** and **13d** are disposed on a human body and are configured to detect respectively a plurality of pieces of motion information **S1**, **S2**, **S3** and **S4**. One pressure sensor **15** is disposed at a plantar aspect of the human body and configured to detect pressure information **P1**. The transceiver **111** is configured to receive the motion information **S1∼S4** of all the motion sensors **13a** to **13d** and the pressure information **P1** of the pressure sensor **15.** Thereafter, the processor **113** determines cycling-posture type information **N1** according to the motion information **S1** to **S4** and the pressure information **P1** and on the basis of a cycling-posture identification model **M1**.

It shall be appreciated that, the electronic device **11** may be a mobile device, a smart device, a smart watch, a tablet computer, an earphone or an electronic product having the displaying function or the audio function. In an implementation, after determining the cycling-posture type information **N1**, the electronic device **11** may display the cycling-posture type information **N1** on a screen, or provide the cycling-posture type information **N1** to a user via an audio device. In some embodiments, the screen may be the screen of a mobile device or a smart watch, and in some other embodiments, the screen may also be the screen of another electronic device or an independent display, and it may be mounted on a bicycle.

The transceiver **111** is capable of wireless communication, and it may receive wireless signals of the motion sensors **13a** to **13d** and the pressure sensor **15,** e.g., Wi-Fi signals, Bluetooth signals, device-to-device wireless signals or the like, and it may also transmit the cycling-posture type information **N1** to any device requesting data via a wireless network. The processor **113** may be a combination of various processing units, central processing units (CPUs), microprocessors or various computing circuits, and it comprises a register or a memory, and the cycling-posture identification model **M1** processes information in the register or the memory of the processor. In an implementation, the electronic device **11** may comprise a storage (not shown), which is configured to store an original cycling-posture identification model **M1** and collect the cycling-posture type information **N1**. The storage may be a hard disk, a universal serial bus (USB) disk, a secure digital (SD) memory card, a mobile disk or other storage media or circuits.

It shall be appreciated that, the motion sensors **13a** to **13d** may be inertial measurement units (IMUs) which may be disposed, tied or worn at positions such as the waist, the thigh, the shank, or the foot of a human body. Additionally, the motion sensors **13a** to **13d** may be disposed, attached or bonded to cycling pants, cycling clothes or cycling shoes.

The pressure sensor **15** is disposed a plantar aspect of the human body, and it may be configured to sense plantar aspect pressure distribution information, e.g., pressure information at toes, the sole or the heel of the foot. The pressure sensor **15** may be combined with bicycle shoes. In an implementation, the cycling-posture analyzing system has two pressure sensors **15** disposed at the plantar aspect of two feet to measure a plurality of pieces of pressure information and obtain a more accurate analyzing result.

Moreover, the motion sensors **13a** to **13d** and the pressure sensor **15** of **FIG. 1** are all capable of wireless signal transmission, and the numbers and disposing positions thereof are only used for illustration and are not limited thereto. Additionally, the motion sensors **13a** to **13d** and the pressure sensor **15** are only disposed at one of two lower limbs of the human body in the first embodiment, but they may also be disposed at both of the two limbs of the human body, and the design thereof shall be appreciated by those of ordinary skill in the art according to the above description.

It shall be appreciated that, the cycling-posture identification model **M1** is a pre-established identification model which records a plurality of kinds of cycling-posture types and the corresponding limb movements and plantar aspect pressure distribution. The cycling-posture identification model **M1** may be established by collecting a large amount of data accompanied with deep learning methods. Therefore, the cycling-posture identifying system may determine the current cycling-posture type of a rider according to the motion information **S1** to **S4,** the pressure information **P1** and the cycling-posture identification model **M1.**

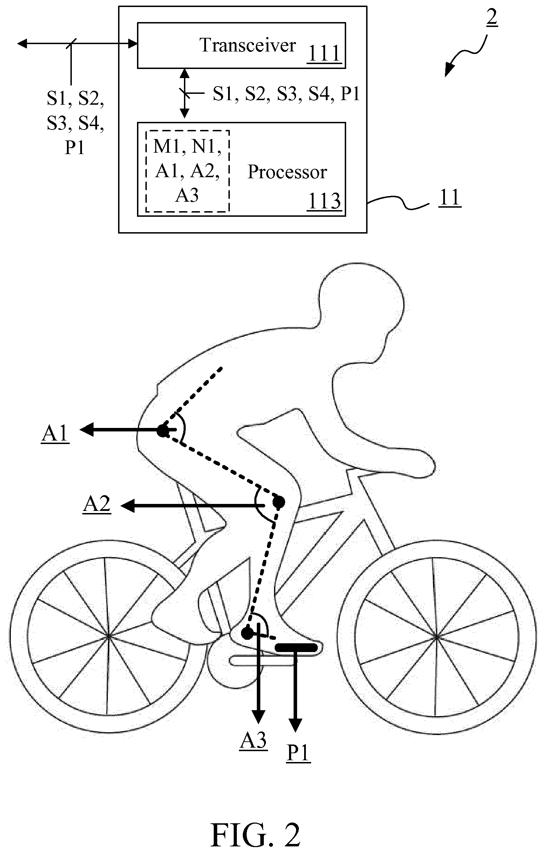

A second embodiment of the present invention is an extension of the first embodiment, and reference is made to **FIG. 1** and **FIG. 2** together for the second embodiment of the present invention. As shown in **FIG. 2****,** elements and functions of the cycling-posture analyzing system **2** disclosed in the second embodiment are similar to these of the cycling-posture analyzing system **1** disclosed in the first embodiment, and thus will not be further described herein, and only differences therebetween will be detailed hereinafter.

In the second embodiment, the processor **113** further calculates at least one piece of angular information according to the motion information **S1** to **S4.** Thereafter, the processor **113** determines the cycling-posture type information **N1** according to the at least one piece of angular information and the pressure information **P1** and on the basis of the cycling-posture identification model **M1**.

In more detail, the processor **113** may calculate a piece of angular information **A1** according to the motion information **S1** and the motion information **S2,** may calculate a piece of angular information **A2** according to the motion information **S2** and the motion information **S3,** and may calculate a piece of angular information **A3** according to the motion information **S3** and the motion information **S4.** The cycling-posture identification model **M1** further records relationships between cycling-posture types and the limb angles as well as plantar aspect pressures. Therefore, the processor **113** may determine the cycling-posture type information **N1** according to at least one of the angular information **A1** to **A3** and the pressure information **P1** and on the basis of the cycling-posture identification model **M1**.

A third embodiment of the present invention is an extension of the second embodiment, and reference is still made to **FIG. 1** and **FIG. 2** for the third embodiment of the present invention. Specifically, the third embodiment is a preferred implementation. The motion sensor **13a** is disposed on a waist of the human body and is configured to detect the motion information **S1** among the plurality of pieces of motion information. The motion sensor **13b** is disposed on a thigh of the human body and is configured to detect the motion information **S2** among the plurality of pieces of motion information. The motion sensor **13c** is disposed on a shank of the human body and is configured to detect the motion information **S3** among the plurality of pieces of motion information. The motion sensor **13d** is disposed on an ankle of the human body and is configured to detect the motion information **S4** among the plurality of pieces of motion information.

The processor **113** calculates the angular information **A1** according to the motion information **S1** and the motion information **S2,** calculates the angular information **A2** according to the motion information **S2** and the motion information **S3,** calculates the angular information **A3** according to the motion information **S3** and the motion information **S4,** and determines the cycling-posture type information **N1** according to the angular information **A1**, the angular information **A2,** the angular information **A3** and the pressure information **P1** and on the basis of the cycling-posture identification model **M1**. In other words, a back-lowering angle, a knee joint angle and an ankle joint angle can be respectively obtained by calculating the angular information **A1** to **A3,** and can be applied to subsequent analysis for analyzing the movement of lower limbs more accurately.

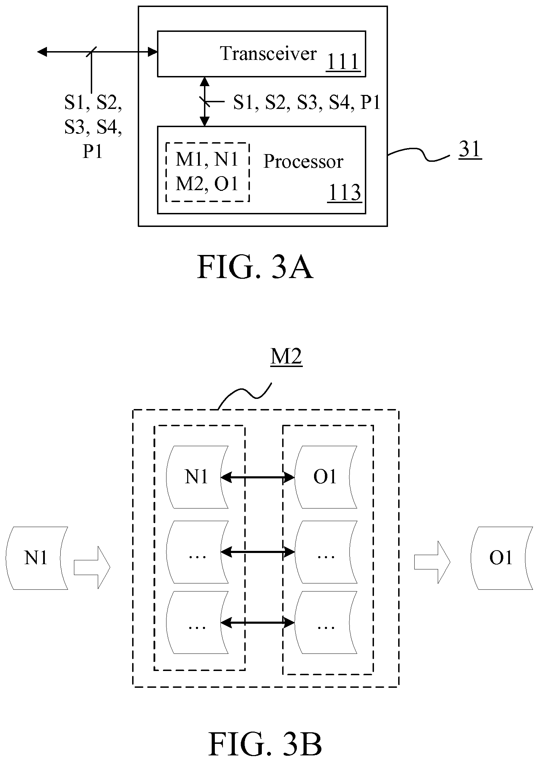

A fourth embodiment of the present invention is an extension of the first embodiment, and reference is made to **FIG. 3A** and **FIG. 3B** for the fourth embodiment of the present invention. **FIG. 3A** is an electronic device **31** of the fourth embodiment, and **FIG. 3B** is a schematic view depicting the operation of a muscle-group usage identification model **M2** according to the fourth embodiment. Elements and functions of the cycling-posture analyzing system of the fourth embodiment are similar to these of the cycling-posture analyzing system of the first embodiment, and thus only differences therebetween will be detailed hereinafter.

Specifically, in the fourth embodiment, the processor **113** further determines muscle-group usage information **O1** according to the cycling-posture type information **N1** and on the basis of a muscle-group usage identification model **M2,** and wherein the muscle-group usage identification model **M2** comprises pre-stored correspondence relationships between the cycling-posture type information and the muscle-group usage information.

In detail, the muscle-group usage identification model **M2** may be established by electromyography experimental data according to laboratories to pre-establish the correspondence relationships between the cycling-posture types and the muscle-group usages. For different cycling-posture types, the hip positions and the cycling-posture types of the rider are all different, and different main muscle groups are applied. The motion information **S1** to **S4** and the pressure information **P1** are first used to analyze the cycling-posture type information **N1**, i.e., the cycling-posture type of the bicycle rider. Thereafter, the current muscle-group usage information **O1** of the rider is further determined according to the predefined muscle-group usage identification model **M2.**

For example, if the cycling-posture type is that the pelvic angle is neutral and natural, then the hip of the rider is located in the middle of the saddle, the cycling state feature is that the pedaling action only involves the stretching of the hip joint and a high-outputting strength can be maintained even after a long period of cycling, and the muscle group mainly used is the gluteus. If the cycling-posture type is that the pelvis tilts forward, then the hip of the rider is located in the front part of the saddle, the cycling state feature is that the angle of the knee joint becomes narrower, which tends to cause the front loading of the handlebars, and the muscle group mainly used is the quadriceps femoris. If the cycling-posture type is that the pelvis tilts backward, then the hip of the rider is located in the back part of the saddle, the cycling state feature is that the femoral joint is stretched using hamstring, one's own weight cannot be used during the pedaling, the pedaling frequency is hard to be increased, and muscle-group mainly used is the hamstring. Therefore, the muscle-group usage identification model **M2** may be established by experimental data of actually measured EMG or sEMG.

In an implementation, the electronic device **31** may comprise a storage (not shown) that is configured to store the cycling-posture identification model **M1,** the muscle-group usage identification model **M2,** the cycling-posture type information **N1** and the muscle-group usage information **O1**.

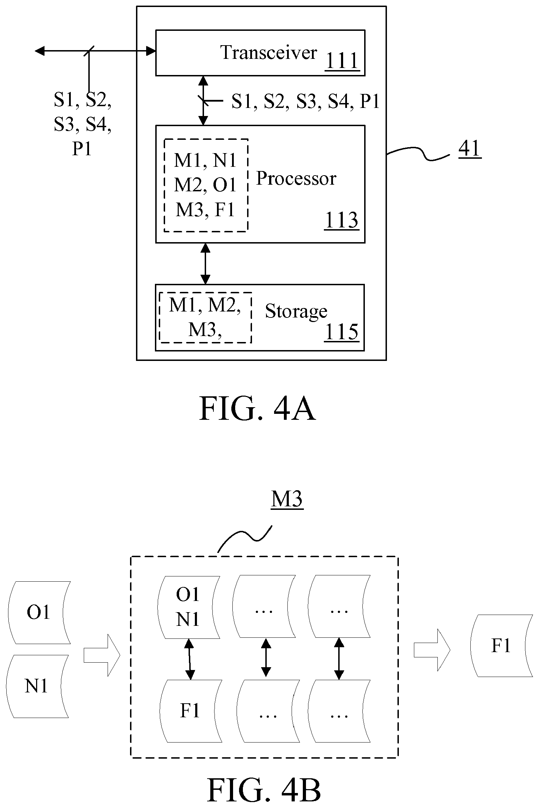

A fifth embodiment of the present invention is an extension of the fourth embodiment, and reference is made to **FIG. 4A** and **FIG. 4B** for the fifth embodiment of the present invention. **FIG. 4A** is an electronic device **41** of the fifth embodiment, and **FIG. 4B** is a schematic view depicting the usage of a feedback model **M3** according to the fourth embodiment. Elements and functions of the cycling-posture analyzing system of the fifth embodiment are similar to these of the cycling-posture analyzing system of the fourth embodiment, and thus only differences therebetween will be detailed hereinafter.

In the fifth embodiment, the processor **113** is further configured to use the cycling-posture type information **N1** and the muscle-group usage information **O1** to decide feedback information **F1** according to a feedback model **M3,** wherein the feedback information **F1** is a piece of cycling-posture type suggestion information. In other words, the cycling-posture analyzing system may further provide a suitable cycling-posture to the rider according to the cycling-posture type information **N1**, the muscle-group usage information **O1** and the feedback model **M3,** thereby prompting the rider to change the cycling-posture type. It shall be appreciated that, the feedback model **M3** is pre-established in the cycling-posture analyzing system, and it comprises correspondence relationships between the cycling-posture type information and the muscle-group usage information and the feedback information.

In an implementation, the electronic device **41** further comprises a storage **115** which is electrically connected to the processor **113** and is configured to store one or a combination thereof. Moreover, the storage **115** may further comprise a database which is configured to store relevant data of the cycling-posture identification model **M1,** the muscle-group usage identification model **M2** and the feedback model **M3** so that the cycling-posture identifying system updates the cycling-posture identification model **M1,** the muscle-group usage identification model **M2** and the feedback model **M3,** or updates the models by machine learning.

In an implementation, the cycling-posture type suggestion information is one of or a combination of cycling-posture type, cycling-posture type suggestion (i.e., suggestions for adjusting the cycling-posture), muscle-group usage suggestion (i.e., suggestions for focusing on the usage of specific muscle-group or suggestions for training another muscle-group) and hip to saddle position suggestion (i.e., suggestions for moving rider's hip from a position of the saddle to another position of the saddle). In other words, the cycling-posture type suggestion information may comprise suggestion data such as the suggested cycling-posture, the pedaling frequency, the pedaling force, the pedaling power, the time of duration, the center-of-gravity position of foot, the joint torque or the like.

In an implementation, the feedback model **M3** has a plurality of kinds of cycling-posture type data and a suggestion rule. The feedback model **M3** may decide that the cycling-posture type of the rider corresponds to one of the plurality kinds of the cycling-posture type data according to the cycling-posture type information **N1** and the muscle-group usage information **O1**, the suggestion rule is configured to record at least one piece of cycling-posture suggestion information corresponding to each of the muscle-group usage information and a usage time of the muscle-group usage information, and the at least one piece of cycling-posture suggestion information is another kind one of the plurality kinds of the cycling-posture type data. In other words, after determining the cycling-posture type data corresponding to the current cycling-posture type, the feedback model **M3** further decides another kind cycling-posture type data that is suitable for the subsequent cycling-posture type according to the muscle-group usage information **O1** and the usage time of the muscle-group usage information **O1**, thereby providing a suggestion of changing the cycling-posture type to the user.

In an implementation, the establishment of the suggestion rule of the feedback model **M3** is achieved by: (1) integrating multiple expert interviews and suggestions given on ordinary textbooks; (2) using expert assistance, analyzing a large amount of user data and machine learning; (3) calculating an ordinary optimal suggestion rule by the knowledge of anatomy and the principle of sport biomechanics, and establishing a suggestion rule customizing a feedback model based on the feedback after each cycling of the user via a method of machine learning; (4) making statistics on cycling strategies used by professional riders in long distance cycling by collecting a large amount of cycling data of multiple professional riders, and establishing a suggestion rule of a feedback model using machine learning; or (5) a combination of the aforesaid four parts.

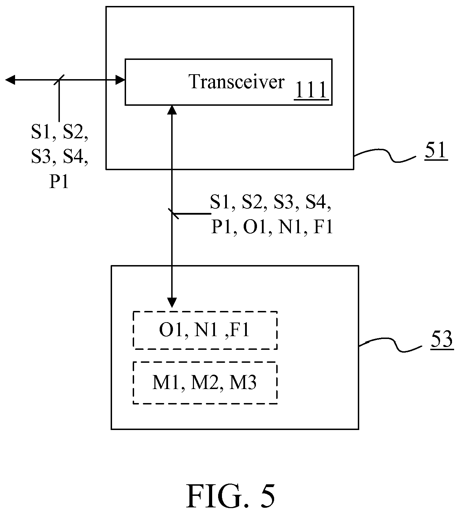

Please refer to **FIG. 5** for a sixth embodiment of the present invention. **FIG. 5** is a schematic view depicting an electronic device **51** of a cycling-posture analyzing system. Referring to **FIG. 5****,** the sixth embodiment is an improvement of the fifth embodiment. Elements and functions of the cycling-posture analyzing system of the sixth embodiment are similar to these of the cycling-posture analyzing system of the fifth embodiment, and thus will not be further described herein, and only differences therebetween will be detailed hereinafter.

In the sixth embodiment, the electronic device **51** comprises a transceiver **111** which is connected with a cloud computing system **53** via a wireless network. The transceiver **111** is configured to receive the motion information **S1** to **S4** from the motion sensors **13a** to **13d** and the pressure information **P1** from the pressure sensor **15.** Thereafter, the transceiver **111** transmits the motion information **S1** to **S4** and the pressure information **P1** to the cloud computing system **53** so that the cloud computing system **53** determines the cycling-posture type information **N1**, the muscle-group usage information **O1** and the feedback information **F1** according to the motion information **S1** to **S4** and the pressure information **PI;** and receive the cycling-posture type information **N1**, the muscle-group usage information **O1** and the feedback information **F1** from the cloud computing system **53.**

More specifically, the electronic device **51** transmits the measured data to the cloud computing system **53** via the transceiver **111** without requiring the processor **113** for operation. The cloud computing system **53** stores the cycling-posture identification model **M1,** the muscle-group usage identification model **M2** and the feedback model **M3.** The cloud computing system **53** may determine the cycling-posture type information **N1** according to the motion information **S1** to **S4** and the pressure information **P1** and on the basis of the cycling-posture identification model **M1**. The cloud computing system **53** may determine the muscle-group usage information **O1** according to the cycling-posture type information **N1** and on the basis of the muscle-group usage identification model **M2.** Thereafter, the cloud computing system **53** may decide the feedback information **F1** according to the muscle-group usage information **O1** and on the basis of the feedback model **M3.**

Moreover, in an implementation, the electronic device **51** may provide the cycling-posture type information **N1**, the muscle-group usage information **O1** and/or the feedback information **F1** to the user via a display device and/or an audio device. Additionally, in another implementation, the cloud computing system **53** may be a system having the operation function, such as a smart phone, a tablet computer or the like.

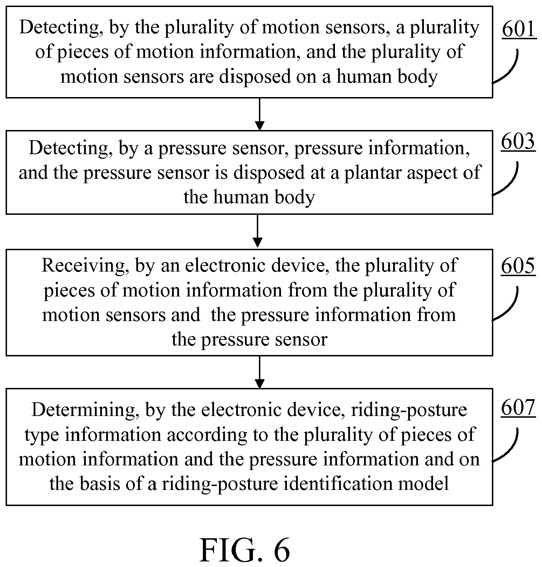

A seventh embodiment of the present invention is a cycling-posture analyzing method, and reference may be made to **FIG. 6** for a flowchart diagram thereof. The method of the seventh embodiment is used for a cycling-posture analyzing system (e.g., the cycling-posture analyzing system of the aforesaid embodiments). The cycling-posture analyzing system comprises an electronic device, a plurality of motion sensors and a pressure sensor. The detailed steps of the seventh embodiment are as follows.

First, step **601** is executed to detect, by the plurality of motion sensors, a plurality of pieces of motion information, and the plurality of motion sensors are disposed on a human body. Step **603** is executed to detect, by the pressure sensor, pressure information, and the pressure sensor is disposed at a plantar aspect of the human body. Step **605** is executed to receive, by the electronic device, the plurality of pieces of motion information from the plurality of motion sensors and the pressure information from the pressure sensor. Step **607** is executed to determine, by the electronic device, cycling-posture type information according to the plurality of pieces of motion information and the pressure information and on the basis of a cycling-posture identification model.

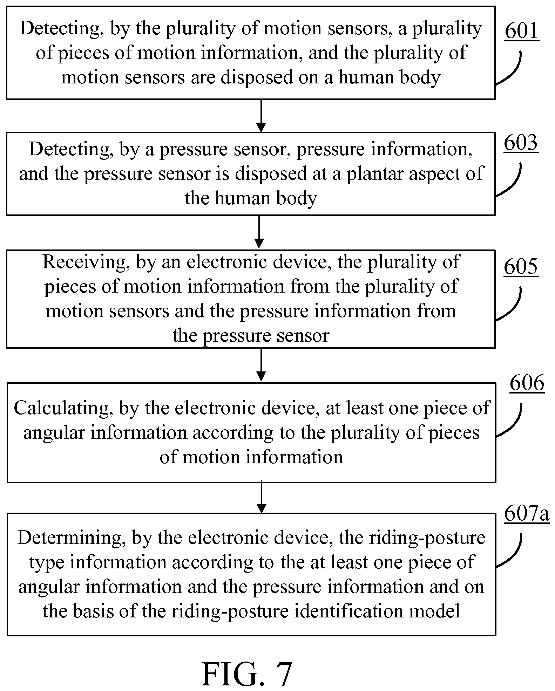

Please refer to **FIG. 7** for an eighth embodiment of the present invention. The steps **601, 603** and **605** are similar to those of the seventh embodiment, and thus will not be further described herein.

After the step **605,** the eighth embodiment further comprises step **606** to calculate, by the electronic device, at least one piece of angular information according to the plurality of pieces of motion information. Thereafter, step **607** further comprises step **607a** to determine, by the electronic device, the cycling-posture type information according to the at least one piece of angular information and the pressure information and on the basis of the cycling-posture identification model.

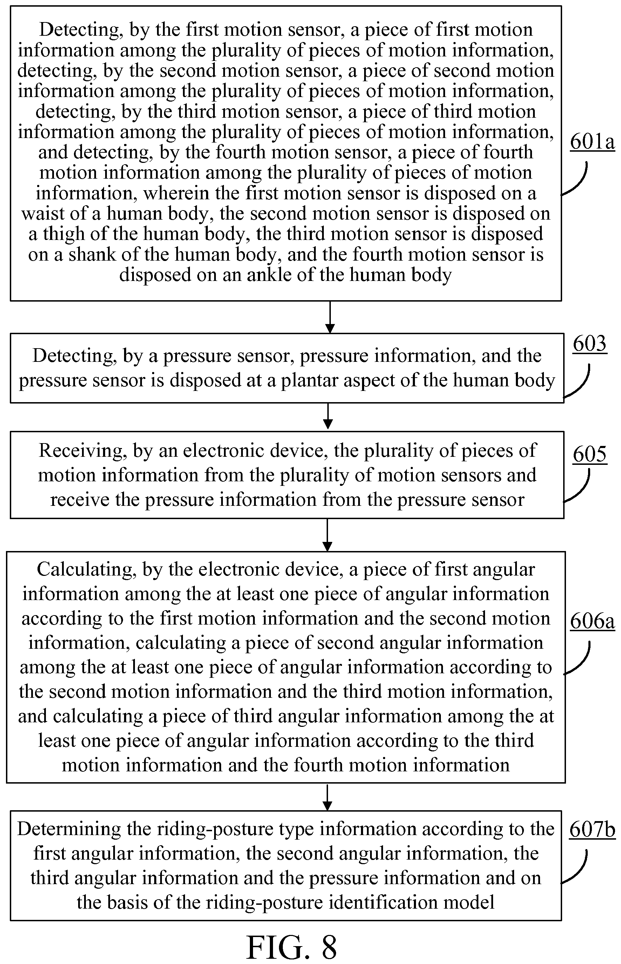

Please refer to **FIG. 8** for a ninth embodiment of the present invention. In this implementation, the plurality of motion sensors further include a first motion sensor, a second motion sensor, a third motion sensor and a fourth motion sensor, wherein the first motion sensor is disposed on a waist of a human body, the second motion sensor is disposed on a thigh of the human body, the third motion sensor is disposed on a shank of the human body, and the fourth motion sensor is disposed on an ankle of the human body. Step **601a** of detecting motion information detects, by the first motion sensor, a piece of first motion information among the plurality of pieces of motion information, detects, by the second motion sensor, a piece of second motion information among the plurality of pieces of motion information, detects, by the third motion sensor, a piece of third motion information among the plurality of pieces of motion information, and detects, by the fourth motion sensor, a piece of fourth motion information among the plurality of pieces of motion information. In some other embodiments, for example, the first motion sensor may be disposed on a waist of a human body, the second motion sensor may be disposed on a thigh of the human body, the third motion sensor may be disposed on a shank of the human body, and the fourth motion sensor may be disposed on an ankle of the human body. Step **603** is executed to detect, by the pressure sensor, pressure information, wherein the pressure sensor is disposed at a plantar aspect of the human body. Step **605** is executed to receive, by the electronic device, the plurality of pieces of motion information from the plurality of motion sensors and the pressure information from the pressure sensor.

Thereafter, the step of calculating at least one piece of angular information further comprises step **606a** of: calculating, by the electronic device, a piece of first angular information among the at least one piece of angular information according to the first motion information and the second motion information, calculating, by the electronic device, a piece of second angular information among the at least one piece of angular information according to the second motion information and the third motion information, and calculating, by the electronic device, a piece of third angular information among the at least one piece of angular information according to the third motion information and the fourth motion information.

The step **607a** further comprises step **607b** of determining the cycling-posture type information according to the first angular information, the second angular information, the third angular information and the pressure information and on the basis of the cycling-posture identification model.

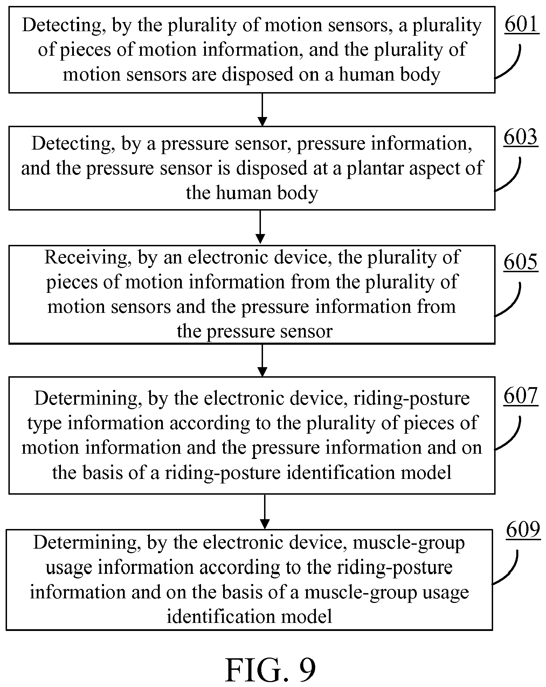

Please refer to **FIG. 9** for a tenth embodiment of the present invention, which is an extension of the seventh embodiment. The flow process of the tenth embodiment is similar to that of the seventh embodiment, and thus the same contents will not be repeated herein and the following description will focus on the differences therebetween.

Specifically, after the step **607,** the cycling-posture analyzing method further comprises step **609** of determining, by the electronic device, muscle-group usage information according to the cycling-posture type information and on the basis of a muscle-group usage identification model.

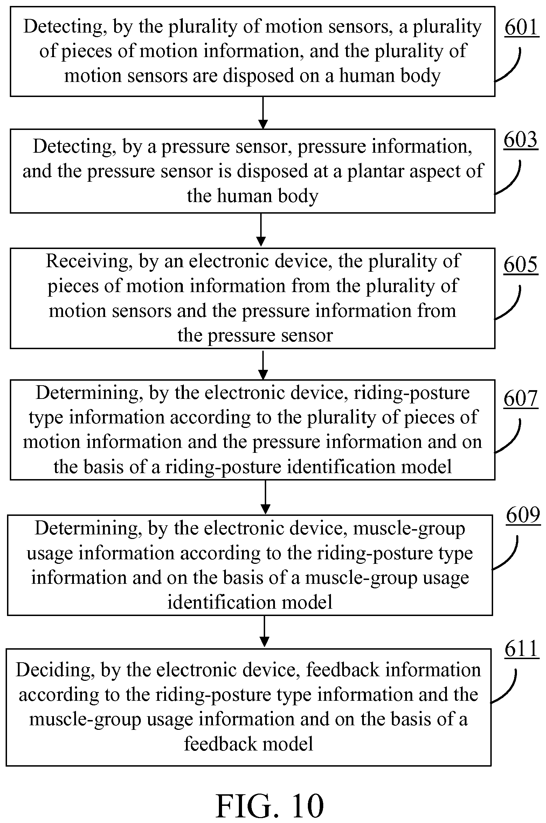

Please refer to **FIG. 10** for an eleventh embodiment of the present invention, which is an extension of the tenth embodiment. The flow process of the eleventh embodiment is similar to that of the tenth embodiment, and thus the same contents will not be repeated herein and the following description will focus on the differences therebetween.

Specifically, after the step **609,** the cycling-posture analyzing method further comprises step **611** of deciding, by the electronic device, feedback information according to the cycling-posture type information and the muscle-group usage information and on the basis of a feedback model.

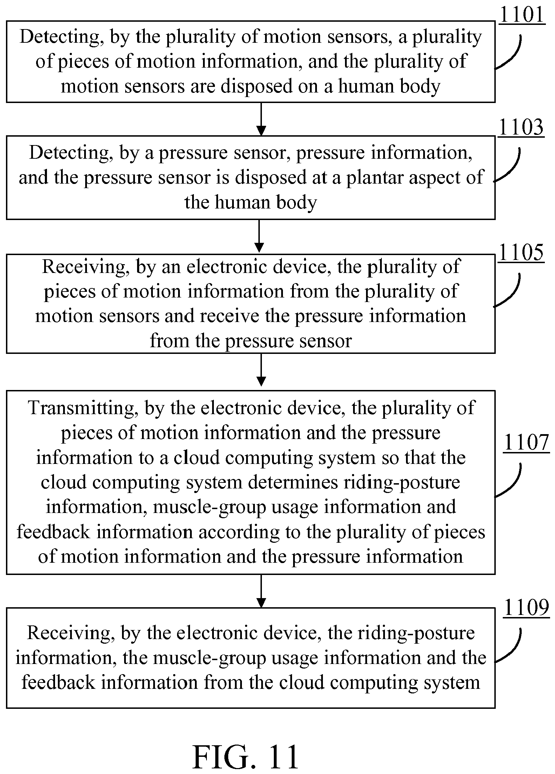

A twelfth embodiment of the present invention is a cycling-posture analyzing method, and reference may be made to **FIG. 11** for a flowchart diagram thereof. The method of the twelfth embodiment is used for a cycling-posture analyzing system (e.g., the cycling-posture analyzing system of the aforesaid embodiments). The cycling-posture analyzing system comprises an electronic device, a plurality of motion sensors and a pressure sensor. The detailed steps of the twelfth embodiment are as follows.

First, step **1101** is executed to detect, by the plurality of motion sensors, a plurality of pieces of motion information, and the plurality of motion sensors are disposed on a human body. Step **1103** is executed to detect, by the pressure sensor, pressure information, and the pressure sensor is disposed at a plantar aspect of the human body. Step **1105** is executed to receive, by the electronic device, the plurality of pieces of motion information from the plurality of motion sensors and the pressure information from the pressure sensor.

Step **1107** is executed to transmit, by the electronic device, the plurality of pieces of motion information and the pressure information to a cloud computing system so that the cloud computing system determines cycling-posture type information, muscle-group usage information and feedback information according to the plurality of pieces of motion information and the pressure information. Step **1107** is executed to receive, by the electronic device, to receive the cycling-posture type information, the muscle-group usage information and the feedback information from the cloud computing system.

In addition to the aforesaid seventh embodiment to twelfth embodiment, the cycling-posture analyzing method of the present invention can also execute all the functions of the cycling-posture analyzing system of the first embodiment to the sixth embodiment of the present invention and achieve the same technical effects, and this will not be further described herein. Moreover, the aforesaid embodiments and implementations can be combined into one embodiment if the technical contents thereof are not conflicting with each other.

As can be known from the above descriptions, the cycling-posture analyzing system and the cycling-posture analyzing method provided according to the present invention perform detection and then determine the cycling-posture type information of the rider according to the motion information and the pressure information and on the basis of the cycling-posture identification model. Moreover, the system and the method can further provide the user with the muscle-group usage information and the feedback information based on the muscle-group usage identification model and the feedback model. The rider can receive the cycling-posture suggestion and accordingly adjust the current cycling-posture type during the cycling. Therefore, as compared to the prior art, the present invention can more effectively provide the rider with relevant cycling information during the outdoor cycling.

The above disclosure is related to the detailed technical contents and inventive features thereof. People skilled in this field may proceed with a variety of modifications and replacements based on the disclosures and suggestions of the invention as described without departing from the characteristics thereof. Nevertheless, although such modifications and replacements are not fully disclosed in the above descriptions, they have substantially been covered in the following claims as appended.

## Claims

1. A cycling-posture analyzing system (1), comprising:
a plurality of motion sensors (13a, 13b, 13c, 13d) disposed on a human body, being configured to detect a plurality of pieces of motion information (S1, S2, S3, S4);
a pressure sensor (15) disposed at a plantar aspect of the human body, being configured to detect pressure information (P1); and
an electronic device (11), comprising:
a transceiver (111) configured to receive the plurality of pieces of motion information (S1, S2, S3, S4) from the plurality of motion sensors (13a, 13b, 13c, 13d) and the pressure information (P1) from the pressure sensor (15); and
a processor (113) electrically connected to the transceiver (111), being configured to determine cycling-posture type information (N1) according to the plurality of pieces of motion information (S1, S2, S3, S4) and the pressure information (P1) and on the basis of a cycling-posture identification model (M1).

2. The cycling-posture analyzing system (2) of Claim 1, wherein the processor (113) is further configured to:
calculate at least one piece of angular information (A1, A2, A3) according to the plurality of pieces of motion information (S1, S2, S3, S4);
determine the cycling-posture type information (N1) according to the at least one piece of angular information (A1, A2, A3) and the pressure information (P1) and on the basis of the cycling-posture identification model (M1).

3. The cycling-posture analyzing system (2) of Claim 2, wherein the plurality of motion sensors (13a, 13b, 13c, 13d) include:
a first motion sensor (13a) disposed on a waist of the human body, configured to detect a piece of first motion information (S1) among the plurality of pieces of motion information (S1, S2, S3, S4);
a second motion sensor (13b) disposed on a thigh of the human body, configured to detect a piece of second motion information (S2) among the plurality of pieces of motion information (S1, S2, S3, S4);
a third motion sensor (13c) disposed on a shank of the human body, configured to detect a piece of third motion information (S3) among the plurality of pieces of motion information (S1, S2, S3, S4); and
a fourth motion sensor (13d) disposed on an ankle of the human body, configured to detect a piece of fourth motion information (S4) among the plurality of pieces of motion information (S1, S2, S3, S4);
wherein the processor (113) is further configured to:
calculate a piece of first angular information (A1) among the at least one piece of angular information (A1, A2, A3) according to the first motion information (S1) and the second motion information (S2);
calculate a piece of second angular information (A2) among the at least one piece of angular information (A1, A2, A3) according to the second motion information (S2) and the third motion information (S3);
calculate a piece of third angular information (A3) among the at least one piece of angular information (A1, A2, A3) according to the third motion information (S3) and the fourth motion information (S4); and
determine the cycling-posture type information (N1) according to the first angular information (A1), the second angular information (A2), the third angular information (A3) and the pressure information (P1) and on the basis of the cycling-posture identification model (M1).

4. The cycling-posture analyzing system of any of Claims 1 to 3, wherein the processor (113) is further configured to:
determine muscle-group usage information (O1) according to the cycling-posture type information (N1) and on the basis of a muscle-group usage identification model (M2).

5. The cycling-posture analyzing system of Claim 4, wherein the processor (113) is further configured to:
decide feedback information (F1) according to the cycling-posture type information (N1) and the muscle-group usage information (O1) and on the basis of a feedback model (M3), wherein the feedback information (F1) shows a piece of cycling-posture type suggestion information.

6. The cycling-posture analyzing system of Claim 5, wherein the electronic device (11) further comprises:
a storage (115) configured to store the cycling-posture identification model (M1), the muscle-group usage identification model (M2) and the feedback model (M3).

7. The cycling-posture analyzing system of Claim 5, wherein the feedback model (M3) has a plurality kinds of cycling-posture type data and a suggestion rule, the cycling-posture type information (N1) and the muscle-group usage information (O1) correspond to one of the plurality kinds of cycling-posture type data, the suggestion rule records at least one piece of cycling-posture type suggestion information corresponding to each of the pieces of muscle-group usage information (O1) and a usage time of the piece of muscle-group usage information (O1), and the at least one piece of cycling-posture type suggestion information is another kind of the plurality of cycling-posture type data.

8. The cycling-posture analyzing system of Claim 5, wherein the cycling-posture suggestion information is one of cycling-posture type, cycling-posture type suggestion, muscle-group usage suggestion and saddle-cushion position suggestion.

9. A cycling-posture analyzing method for a cycling-posture analyzing system (1), the cycling-posture analyzing system (1) comprising a plurality of motion sensors (13a, 13b, 13c, 13d), a pressure sensor (15) and an electronic device (11), and the cycling-posture analyzing method comprising:
detecting, by the plurality of motion sensors (13a, 13b, 13c, 13d), a plurality of pieces of motion information (S1, S2, S3, S4), the plurality of motion sensors (13a, 13b, 13c, 13d) being disposed on a human body;
detecting, by the pressure sensor (15), pressure information (PI), the pressure sensor (15) being disposed at a plantar aspect of the human body;
receiving, by the electronic device (11), the plurality of pieces of motion information (S1, S2, S3, S4) from the plurality of motion sensors (13a, 13b, 13c, 13d) and receive the pressure information (P1) from the pressure sensor (15); and
determining, by the electronic device (11), cycling-posture type information (N1) and on the basis of the plurality of pieces of motion information (S1, S2, S3, S4) and the pressure information (P1) and on the basis of a cycling-posture identification model (M1).

10. The cycling-posture analyzing method of Claim 9, further comprising:
calculating, by the electronic device (11), at least one piece of angular information (A1, A2, A3) according to the plurality of pieces of motion information (S1, S2, S3, S4);
determining, by the electronic device (11), the cycling-posture type information (N1) and on the basis of the at least one piece of angular information (A1, A2, A3) and the pressure information (P1) and on the basis of the cycling-posture identification model (M1).

11. The cycling-posture analyzing method of Claim 10, wherein the plurality of motion sensors (13a, 13b, 13c, 13d) further include a first motion sensor (13a), a second motion sensor (13b), a third motion sensor (13c) and a fourth motion sensor (13d), and the step of detecting the plurality of pieces of motion information (S1, S2, S3, S4) further comprises:
detecting, by the first motion sensor (13a), a piece of first motion information (S1) among the plurality of pieces of motion information (S1, S2, S3, S4), the first motion sensor (13a) being disposed on a waist of the human body;
detecting, by the second motion sensor (13b), a piece of second motion information (S2) among the plurality of pieces of motion information (S1, S2, S3, S4), the second motion sensor (13b) being disposed on a thigh of the human body;
detecting, by the third motion sensor (13c), a piece of third motion information (S3) among the plurality of pieces of motion information (S1, S2, S3, S4), the third motion sensor (13c) being disposed on a shank of the human body; and
detecting, by the fourth motion sensor (13d), a piece of fourth motion information (S4) among the plurality of pieces of motion information (S1, S2, S3, S4), the fourth motion sensor (13d) being disposed on an ankle of the human body;
wherein the step of calculating the at least one piece of angular information (A1, A2, A3) further comprises:
calculating, by the electronic device (11), a piece of first angular information (A1) among the at least one piece of angular information (A1, A2, A3) according to the first motion information (S1) and the second motion information (S2);
calculating, by the electronic device (11), a piece of second angular information (A2) among the at least one piece of angular information (A1, A2, A3) according to the second motion information (S2) and the third motion information (S3);
calculating, by the electronic device (11), a piece of third angular information (A3) among the at least one piece of angular information (A1, A2, A3) according to the third motion information (S3) and the fourth motion information (S4); and
determining, by the electronic device (11), the cycling-posture type information (N1) according to the first angular information (A1), the second angular information (A2), the third angular information (A3) and the pressure information (P1) and on the basis of the cycling-posture identification model (M1).

12. The cycling-posture analyzing method of any of Claims 9 to 11, further comprising:
determining, by the electronic device (11), muscle-group usage information (O1) according to the cycling-posture type information (N1) and on the basis of a muscle-group usage identification model (M2).

13. The cycling-posture analyzing method of Claim 12, further comprising:
deciding, by the electronic device (11), feedback information (F1) according to the cycling-posture type information (N1) and the muscle-group usage information (O1) and on the basis of a feedback model (M3), wherein the feedback information (F1) shows a suggestion for cycling-posture.

14. The cycling-posture analyzing method of Claim 13, wherein the feedback model (M3) has a plurality kinds of cycling-posture type data and a suggestion rule, the cycling-posture type information (N1) and the muscle-group usage information (O1) correspond to one of the plurality kinds of cycling-posture type data, the suggestion rule records at least one piece of cycling-posture suggestion information corresponding to each of the pieces of muscle-group usage information (O1) and a usage time of the piece of muscle-group usage information (O1), and the at least one piece of cycling-posture suggestion information is another kind of the plurality of cycling-posture type data.

15. The cycling-posture analyzing method of Claim 13, wherein the cycling-posture suggestion information is one of cycling-posture type, cycling-posture type suggestion, muscle-group usage suggestion and saddle-cushion position suggestion.
